# EUROPEAN PATENT SPECIFICATION

(11) **EP 2 344 161 B1**
(45) Date of publication and mention of the grant of the patent: **19.12.2018**
(21) Application number: 09820164.3
(22) Date of filing: 16.10.2009
(51) Int. Cl.: A61K 31/4745, A61K 31/496, A61K 31/513, A61K 31/7072, A61K 39/395, A61K 9/127, A61P 35/00

(54) **COMBINATIONS OF A LIPOSOMAL WATER-SOLUBLE CAMPTOTHECIN WITH CETUXIMAB OR BEVACIZUMAB**
KOMBINATIONEN EINES LIPOSOMALEN WASSERLÖSLICHEN CAMPTOTHECINS MIT CETUXIMAB ODER BEVACIZUMAB
COMBINAISONS D'UNE CAMPTOTHÉCINE DE LIPOSOME SOLUBLE DANS L'EAU AVEC DU CETUXIMAB OU DU BEVACIZUMAB

(30) Priority: 16.10.2008 US 106109 P
(43) Date of publication of application: 20.07.2011
(73) Proprietor: Celator Pharmaceuticals, Inc., Ewing, NJ 08628 (US)
(72) Inventor: TARDI, Paul, Surrey, British Columbia V3S 1S4 (CA); MAYER, Lawrence, North Vancouver British Columbia V7J 3M4 (CA); BERMUDES, David, Kenwood CA 95452 (US)
(74) Representative: V.O.
(86) International application number: PCT/CA2009/001483
(87) International publication number: WO 2010/043050

(56) References cited:
- WO-A1-03/030864
- WO-A1-2005/002546
- WO-A1-2005/117877
- WO-A2-2004/087115
- WO-A2-2004/087115
- WO-A2-2009/024667
- S. SATHORNSUMETEE ET AL: "Tumor Angiogenic and Hypoxic Profiles Predict Radiographic Response and Survival in Malignant Astrocytoma Patients Treated With Bevacizumab and Irinotecan", JOURNAL OF CLINICAL ONCOLOGY, vol. 26, no. 2, 10 January 2008 (2008-01-10), pages 271-278, XP055121262, ISSN: 0732-183X, DOI: 10.1200/JCO.2007.13.3652
- KIM ET AL.: 'Cetuximab and irinotecan interact synergistically to inhibit the growth of orthotopic anaplastic thyroid carcinoma xenografts in nude mice' CLIN. CANCER RES. vol. 12, no. 2, 2006, pages 600 - 607, XP008136963
- MAHTANI ET AL.: 'Synergy between cetuximab and chemotherapy in tumors of the gastrointestinal tract' ONCOLOGIST vol. 13, 2008, pages 39 - 50, XP008137142
- OOYAMA A ET AL: "Anti-angiogenic effect of 5-Fluorouracil-based drugs against human colon cancer xenografts", CANCER LETTERS, NEW YORK, NY, US, vol. 267, no. 1, 18 August 2008 (2008-08-18), pages 26-36, XP022797039, ISSN: 0304-3835, DOI: 10.1016/J.CANLET.2008.03.008 [retrieved on 2008-04-16]

## Description

### Technical Field

The invention relates to combinations of targeted antitumor agents that exhibit enhanced effects against hyperproliferative conditions.

### Background Art

The progression of many life-threatening diseases such as cancer, AIDS, infectious diseases, immune disorders and cardiovascular disorders is influenced by multiple molecular mechanisms. Due to this complexity, achieving cures with a single agent has been met with limited success. Thus, combinations of agents have often been used to combat disease, particularly in the treatment of cancers. It appears that there is a strong correlation between the number of agents administered and cure rates for cancers such as acute lymphocytic leukemia and metastatic colorectal cancer (Frei, et al., Clin. Cancer Res. (1998) 4:2027-2037; Fisher, M. D., Clin Colorectal Cancer (2001) Aug;1(2):85-86). In particular, chemotherapeutic agents (e.g. camptothecins) in combination with other targeted antitumor agents, such as those that inhibit angiogenesis or that target/decrease a protein or lipid kinase activity, have been used to successfully treat cancers in the clinic.

Camptothecin is a quinoline-based alkaloid found in the bark of the Chinese camptotheca tree and the Asian nothapodytes tree. Many derivatives of camptothecin including semi-synthetic or synthetic derivatives, such as topotecan and irinotecan, have a unique ability to inhibit topoisomerase I which has made them highly active cell-killing agents. Topoisomerase I is a cellular enzyme responsible for the winding and unwinding of DNA. If the DNA cannot be unwound, then transcription of the DNA message cannot occur and protein will not be synthesized, resulting in the eventual death of the cell. Cells that are dividing at a rapid rate, such as cancer cells, are particularly sensitive to camptothecin derivatives as their DNA is constantly being unwound in order to be replicated for daughter cells. In the open state, the DNA is vulnerable to insertion of camptothecin drugs which has been shown to result in the eventual breaking of the DNA and cell death.

As described herein below, the therapeutic combinations and compositions of the present invention may also include the addition of a fluoropyrimidine such as fluorouracil (5-FU) or floxuridine (FUDR). 5-FU was introduced into clinical trials approximately 40 years ago; it was not until the early 1990's that trials involving combinations of camptothecin derivatives with pyrimidine analogs were investigated (Furuta, T., et al., Gan To Kagaku Ryoho (1991) Mar;18(3):393-402). Promising improvements in cancer treatment were found by administering free drug cocktails of a number of pyrimidine/camptothecin combinations (see PCT publications WO/0066125 and WO/00162235). For example, U.S. patent 6,403,569 claims a method for treating cancer by administering a synergistic amount of a camptothecin derivative, 5-FU, and leucovorin (a compound related to the vitamin folic acid which is a standard practice of care during 5-FU treatment), providing that there is at least 200 mg/m² of leucovorin.

Despite the advantages associated with the use of pyrimidine/camptothecin drug cocktails, there are various drawbacks that limit their therapeutic use. For instance, administration of free drug cocktails often results in rapid clearance of one or all of the drugs before reaching the tumor site. For this reason, many drugs have been incorporated into delivery vehicles designed to 'shield' them from mechanisms that would otherwise result in their clearance from the bloodstream. It is known that liposomes have the ability to provide this 'shielding' effect and they are thus, able to extend the half-life of therapeutic agents.

Encapsulation of drugs into well-designed delivery vehicles can also result in coordinated pharmacokinetics of encapsulated drugs. The present inventors have identified particular delivery vehicle formulations required to accommodate a combination of pyrimidine and camptothecin derivatives. PCT publication WO03/028696, assigned to Celator Pharmaceuticals, describes compositions and methods of administering non-antagonistic mol ratios of two or more biologically active agents stably associated with delivery vehicles such as liposomes, such that the favorable mol ratios are maintained after administration to a subject. PCT publication WO2004/087115 describes particular embodiments of such compositions wherein the liposomes contain at least one camptothecin and at least one fluoropyrimidine. A particular embodiment of these agents is described and has become known as CPX-1. This particular embodiment has had success in clinical trials.

Kim et al., Clin. Cancer Res. (2006) 12(2):600-607 discloses that Cetuximab® and irinotecan interact synergistically to inhibit the growth of orthotopic anaplastic thyroid carcinoma xenografts in nude mice and that such combination of agents provides for an improved treatment of irinotecan-refractory colorectal cancer.

It is now found that supplementing such liposomal compositions as described above with additional targeted antitumor agents enhances their therapeutic effect. This is surprising, since it was thought that the presence of these targeted antitumor agents would inhibit the uptake of liposomes from the vasculature and thus, partially nullify the effect of the liposomal formulation.

### Disclosure of the Invention

The combinations and compositions underlying the present invention are useful for treating hyperproliferative diseases. Hyperproliferative diseases are generally cancer and/or any metastases. Combinations and compositions underlying the present invention are particularly useful for treating colorectal tumors.

Embodiments of the combinations and compositions underlying the present invention allow for the administration of effective amounts of an antiangiogenic targeted antitumor agent along with fluoropyrimidine/camptothecin drug combinations using liposomal vehicles that are stably associated with at least one fluoropyrimidine and one water-soluble camptothecin at a non-antagonistic ratio. (In other embodiments, only liposomal camptothecin and the targeted antitumor agent are used.) The liposomal camptothecin/fluoropyrimidine compositions allow the camptothecin and fluoropyrimidine to be delivered to the disease site in a coordinated fashion, thereby assuring that these agents will be present at the disease site at a desired ratio. This result will be achieved whether the agents are co-encapsulated in liposomes, or are separately encapsulated and administered such that desired ratios are maintained at the disease site. The pharmacokinetics (PK) of the composition are controlled by the liposomes themselves such that coordinated delivery is achieved (provided that the PK of the delivery systems are comparable).

Thus, in one aspect, the invention is directed to a combination of
a) first liposomes stably associated with at least one water-soluble camptothecin; and
b) an antiangiogenic targeted antitumor agent which is a compound that decreases or inhibits the activity of an epidermal growth factor family receptor (EGFR) tyrosine kinase or that inhibits the activity of a vascular endothelial growth factor (VEGF) receptor tyrosine kinase or that binds to VEGF;
for use in treating a cancer in a subject. In preferred embodiments liposomes are stably associated with a fluoropyrimidine and with said camptothecin, wherein the mol ratio of the camptothecin to the fluoropyrimidine is non-antagonistic. In one such embodiment, said first liposomes further comprise a fluoropyrimidine, wherein the mol ratio of said camptothecin to said fluoropyrimidine is non-antagonistic, and the camptothecin and fluoropyrimidine are stably associated with said first liposomes. In another embodiment, the combination further includes a fluoropyrimidine stably associated with second liposomes, wherein the mol ratio of said fluoropyrimidine and said water-soluble camptothecin is non-antagonistic, and the pharmacokinetics of said first and second liposomes are coordinated.

In other aspects, the invention is directed to a composition comprising
(a) liposomes associated with at least one water-soluble camptothecin; and
(b) an antiangiogenic targeted antitumor agent which is a compound that decreases or inhibits the activity of an epidermal growth factor family receptor (EGFR) tyrosine kinase or that inhibits the activity of a vascular endothelial growth factor (VEGF) receptor tyrosine kinase or that binds to VEGF;
for use in treating a cancer in a subject.

As further described below, in a preferred embodiment, in designing an appropriate combination to include a liposomal water-soluble camptothecin and a liposomal fluoropyrimidine, the water-soluble camptothecin and fluoropyrimidine are present at a non-antagonistic ratio over a wide concentration range. Methods and criteria for determining this are described in detail in WO03/028696, *supra.* Suitable liposomal formulations are designed such that they stably incorporate an effective amount of a fluoropyrimidine/water-soluble camptothecin combination and allow for the coordinated release of both drugs *in vivo.* This is described in WO03/028696 as well. Preferred formulations contain at least one negatively charged lipid, such as phosphatidylglycerol, and contain at least one sterol, such as cholesterol.

### Brief Description of the Drawings

FIGURE 1A is a graph of the efficacy of Cetuximab® (squares), irinotecan (triangles) or a combination of Cetuximab® and irinotecan (circles) when administered to mice bearing DLD-1 human colon xenografts.
FIGURE 1B is a graph of the efficacy of Cetuximab® (squares), liposomal irinotecan (triangles) or a combination of Cetuximab® and liposomal irinotecan (circles) when administered to mice bearing DLD-1 human colon xenografts.
FIGURE 2A is a graph of the efficacy of bevacizumab (squares), CPX-1 (triangles) or a combination of bevacizumab and CPX-1 administered concurrently (circles) when administered to mice bearing LS174T human colon xenografts.
FIGURE 2B is a graph of the efficacy of bevacizumab (squares), CPX-1 (triangles) or a combination of bevacizumab and CPX-1 when two injections of bevacizumab are given prior to one dose of CPX-1 (circles) to mice bearing LS174T human colon xenografts.

### Modes of Carrying Out the Invention

As noted herein above, the invention provides combinations and compositions for use in treating a cancer wherein an antiangiogenic targeted antitumor agent is to be administered in combination with liposomes stably associated with at least one water-soluble camptothecin, and in some embodiments, in further combination with liposomes stably associated with at least one fluoropyrimidine at a non-antagonistic ratio to the camptothecin.

In embodiments wherein at least one water soluble camptothecin and at least one fluoropyrimidine are "stably associated" with liposomes, "stably associated" means that the ratio of these agents to be administered to a subject is maintained in the blood of a subject for at least one hour after administration. This is in contrast to administration as a free drug cocktail where the ratio will inevitably be altered after administration. Typically, the ratio will not vary by more than 5% over this time.

By a "non-antagonistic" ratio is meant that when this ratio is provided to cancer cells relevant to a cancer in a subject in an *in vitro* assay, the combination is non-antagonistic over a concentration range at which the fraction of affected cells is 0.20-0.80 over at least 20% of this range. In general, for camptothecins and fluoropyrimidines, the appropriate mol ratio is of the order of 1:1.

When combinations of camptothecins and fluoropyrimidines are employed, they may be stably associated with the same liposomes - *i.e.,* co-encapsulated, or may be stably associated with separately prepared liposomes, as long as the pharmacokinetics are controlled in such a way that the ratio is maintained as set forth above.

Thus, in one embodiment, the combination for use in treating a cancer concerns liposomes stably associated with a water-soluble camptothecin which are also to be administered to a subject with an antiangiogenic targeted antitumor agent. The liposomal camptothecin and the targeted antitumor agent may be administered in the same composition, in separate compositions at the same time, or sequentially in any order. Thus, the liposomal camptothecin may be administered first and the targeted antitumor agent second, or *vice versa.* In addition, multiple dosages of each may be provided. Thus, the liposomal camptothecin may be administered first, the targeted antitumor agent second, and then another administration of the liposomal camptothecin following. Any of these dosing events can be repeated as needed. The same number of dosing events for each of the drugs in the combination need not be the same.

Similar comments apply to administration of the combination of camptothecin and fluoropyrimidine stably associated with liposomes and another antiangiogenic targeted antitumor agent. Typically, however, the stably associated camptothecin/fluoropyrimidine composition is co-administered.

While generally, only a single camptothecin and a single fluoropyrimidine are used, mixtures of each of these elements may also be employed. In general, the use of terms such as "a" and "an" may denote one or more than one.

### Water-Soluble Camptothecins

Nearly all naturally occurring camptothecins are poorly water-soluble; this property makes them difficult, and in many instances impossible, to formulate and administer. As a result, many camptothecins marketed or in development have been made water-soluble. Water-soluble camptothecins include those derivatives of camptothecin that are charged at physiological pH. For example, enhanced water-solubility has been effectively achieved through addition of a hydrophilic hydroxyl or nitro group at the 9, 10, or 11 positions of the camptothecin A ring. Similarly, addition of a positively charged dimethylaminomethyl group at the 9 position has demonstrated enhanced water-solubility.

Water-soluble derivatives of camptothecin have shown a broad spectrum of activity against human tumors. Because of this, the United States Food and Drug Administration (FDA) have approved water-soluble camptothecin formulations of irinotecan, topotecan and lurtotecan for clinical use in humans. The antitumor activity demonstrated with irinotecan is thought to occur through its metabolite, SN-38.

"Water-soluble camptothecins" in the context of the present invention refers to derivatives of camptothecin or formulations thereof that are sufficiently soluble in water. Water-soluble camptothecins include, but are not limited to, irinotecan, SN-38, topotecan, 9-aminocamptothecin, lurtotecan and prodrugs, precursors, metabolic products of these drugs; as well as hydrophilic salt derivatives of water-insoluble camptothecins such as the sodium salt of the parent compound, camptothecin. Preferably, the water-soluble camptothecin for use in this invention is irinotecan, topotecan, 9-aminocamptothecin or lurtotecan. Most preferably, the water-soluble camptothecin is irinotecan.

### Antiangionic Targeted Antitumor Agents

"Antiangiogenic targeted antitumor agents" in the context of the present invention refers to compounds targeting, decreasing or inhibiting the activity of an epidermal growth factor family of receptor tyrosine kinases (EGFR, ErbB2, ErbB3, ErbB4 as homo- or heterodimers), or to compounds which inhibit the activity of a vascular endothelial growth factor (VEGF) receptor tyrosine kinase, inhibit a VEGF receptor or bind to VEGF, and are in particular those compounds, proteins or monoclonal antibodies generically and specifically disclosed in WO 98/35958, *e.g*., 1-(4-chloroanilino)-4-(4-pyridylmethyl)phthalazine or a pharmaceutically acceptable salt thereof, *e.g.,* the succinate, or in WO-00/09495, WO-00/27820, WO-00/59509, WO-98/11223, WO-00/27819 and EP 0769947; those as described by Prewett, M., et al., in Cancer Research (1999) 59:5209-5218, by Yuan, F., et al., in Proc. Natl. Acad. Sci. USA (1996) 93:14765-14770, by Zhu, Z., et al., in Cancer Res. (1998) 58:3209-3214, and by Mordenti, J., et al., in Toxicologic Pathology (1999) 27:14-21; in WO 00/37502 and WO 94/10202; Angiostatin™, described by O'Reilly, M. S., et al., Cell (1994) 79:315-328; Endostatin™, described by O'Reilly, M. S., et al., Cell (1997) 88:277-285; anthranilic acid amides; ZD4190; ZD6474; SU5416; SU6668; or anti-VEGF antibodies or anti-VEGF receptor antibodies, *e.g*., RhuMab.

By antibody is meant intact monoclonal antibodies, polyclonal antibodies, multispecific antibodies formed from at least 2 intact antibodies, and antibody fragments so long as they exhibit the desired biological activity. Single chain forms are also included.

Antiangiogenic targeted antitumor agents which target, decrease or inhibit the activity of the epidermal growth factor receptor family are especially compounds, proteins or antibodies which inhibit members of the EGF receptor tyrosine kinase family, *e.g*., EGF receptor, ErbB2, ErbB3 and ErbB4 or bind to EGF or EGF related ligands, and are in particular those compounds, proteins or monoclonal antibodies generically and specifically disclosed in WO 97/02266, *e.g*., the compound of Ex. 39, or in EP 0564409, WO 99/03854, EP 0520722, EP 0566226, EP 0787722, EP 0837063, U.S. Pat. No. 5,747,498, WO 98/10767, WO 97/30034, WO 97/49688, WO 97/38983 and, especially, WO 96/30347 (*e.g.,* compound known as CP 358774), WO 96/33980 (*e.g.,* compound ZD 1839) and WO 95/03283 (*e.g.,* compound ZM105180); *e.g.,* trastuzumab (Herpetin®), Cetuximab®, Iressa®, OSI-774, CI-1033, EKB-569, GW-2016, E1.1, E2.4, E2.5, E6.2, E6.4, E2.11, E6.3 or E7.6.3.

The term cyclooxygenase inhibitor as used herein includes, but is not limited to, *e.g.,* celecoxib (Celebrex®), rofecoxib (Vioxx®), etoricoxib, valdecoxib or a 5-alkyl-2-arylaminophenylacetic acid, *e.g*., 5-methyl-2-(2'-chloro-6'-fluoroanilino)phenyl acetic acid.

The term "bisphosphonates" as used herein includes, but is not limited to, etridonic, clodronic, tiludronic, pamidronic, alendronic, ibandronic, risedronic and zoledronic acid. "Etridonic acid" can be administered, *e.g*., in the form as it is marketed, *e.g.,* under the trademark DIDRONEL™. "Clodronic acid" can be administered, *e.g.,* in the form as it is marketed, *e.g*., under the trademark BONEFOS™. "Tiludronic acid" can be administered, *e.g.,* in the form as it is marketed, *e.g.,* under the trademark SKELID™. "Pamidronic acid" can be administered, *e.g.,* in the form as it is marketed, *e.g.,* under the trademark AREDIA™. "Alendronic acid" can be administered, *e.g*., in the form as it is marketed, *e.g*., under the trademark FOSAMAX™. "Ibandronic acid" can be administered, *e.g.,* in the form as it is marketed, *e.g.,* under the trademark BONDRANAT™. "Risedronic acid" can be administered, *e.g*., in the form as it is marketed, *e.g*., under the trademark ACTONEL™. "Zoledronic acid" can be administered, *e.g.,* in the form as it is marketed, *e.g.,* under the trademark ZOMETA™.

### In vitro Determination of Drug Combination Synergy

In some embodiments of the invention camptothecins and fluoropyrimidines will be encapsulated in liposomes at synergistic or additive (*i.e.,* non-antagonistic) ratios (a composition termed "CPX-1") and are to be administered with a biological agent. Determination of ratios of camptothecins and fluoropyrimidines that display synergistic or additive combination effects may be carried out using various algorithms, based on the types of experimental data as described in PCT publications WO 03/028696 and WO2004/087115 (*supra*).

### Preparation of Lipid-Based Delivery Vehicles for Camptothecins and Fhioropyrimidines

Lipid carriers for use in this invention are liposomes. Liposomes can be prepared as described in Liposomes: Rational Design (A.S. Janoff, ed., Marcel Dekker, Inc., New York, NY), or by additional techniques known to those knowledgeable in the art. Suitable liposomes for use in this invention include large unilamellar vesicles (LUVs), multilamellar vesicles (MLVs), small unilamellar vesicles (SUVs) and interdigitating fusion liposomes.

Liposomes for use in this invention may be prepared to be of "low-cholesterol." Such liposomes allow for the presence of an amount of cholesterol that is insufficient to significantly alter the phase transition characteristics of the liposome (typically less than 20 mol % cholesterol). Liposomes for use in the present invention may also contain therapeutic lipids, examples of which include ether lipids, phosphatidic acid, phosphonates, ceramide and ceramide analogs, sphingosine and sphingosine analogs and serine-containing lipids.

Liposomes may also be prepared with surface stabilizing hydrophilic polymer-lipid conjugates such as polyethylene glycol-DSPE, to enhance circulation longevity. The incorporation of negatively charged lipids such as phosphatidylglycerol (PG) and phosphatidylinositol (PI) may also be added to liposome formulations to increase the circulation longevity of the carrier. These lipids may be employed to replace hydrophilic polymer-lipid conjugates as surface stabilizing agents. Preferred embodiments of this invention may make use of low-cholesterol liposomes containing PG or PI to prevent aggregation, thereby increasing the blood residence time of the carrier.

Various methods may be utilized to encapsulate active agents in liposomes. "Encapsulation," includes covalent or non-covalent association of an agent with the lipid-based delivery vehicle. For example, this can be by interaction of the agent with the outer layer or layers of the liposome or entrapment of an agent within the liposome, equilibrium being achieved between different portions of the liposome. Thus, encapsulation of an agent can be by association of the agent by interaction with the bilayer of the liposomes through covalent or non-covalent interaction with the lipid components or entrapment in the aqueous interior of the liposome, or in equilibrium between the internal aqueous phase and the bilayer.

Encapsulation of a desired combination can be achieved either through encapsulation in separate delivery vehicles or within the same delivery vehicle. Where encapsulation into separate liposomes is desired, the lipid composition of each liposome may be quite different to allow for coordinated pharmacokinetics. By altering the vehicle composition, release rates of encapsulated drugs can be matched to allow desired ratios of the drugs to be delivered to the tumor site. When two or more drugs are encapsulated in separate liposomes, it should be readily accepted that ratios of water-soluble camptothecins to fluoropyrimidines that have been determined on a patient-specific basis to provide optimal therapeutic activity, would be generated for individual patients by combining the appropriate amounts of each liposome-encapsulated drug prior to administration. Alternatively, two or more agents may be encapsulated within the same liposome.

### Administering Compositions underlying the Invention In vivo

As mentioned above, the compositions for use in treating cancer according to the present invention may be administered to warm-blooded animals, including humans, as well as to domestic avian species. For treatment of human ailments, a qualified physician will determine how the compositions underlying the present invention should be utilized with respect to dose, schedule and route of administration using established protocols. Such applications may also utilize dose escalation should agents encapsulated in the liposomal compositions exhibit reduced toxicity to healthy tissues of the subject.

Preferably, the pharmaceutical compositions for use according to the present invention are to be administered parenterally, *i.e.,* intraarterially, intravenously, intraperitoneally, subcutaneously, or intramuscularly. More preferably, the pharmaceutical compositions are to be administered intravenously or intraperitoneally by a bolus injection. However, any effective method of administration may be used, including endoscopic procedures.

Pharmaceutical compositions for use according to the present invention are prepared according to standard techniques and may comprise water, buffered water, 0.9% saline, 0.3% glycine, 5% dextrose and the like, including glycoproteins for enhanced stability, such as albumin, lipoprotein, globulin, and the like. These compositions may be sterilized by conventional, well-known sterilization techniques. The resulting aqueous solutions may be packaged for use or filtered under aseptic conditions and lyophilized, the lyophilized preparation being combined with a sterile aqueous solution prior to administration. The compositions may contain pharmaceutically acceptable auxiliary substances as required to approximate physiological conditions, such as pH adjusting and buffering agents, tonicity adjusting agents and the like, for example, sodium acetate, sodium lactate, sodium chloride, potassium chloride, calcium chloride, and the like. Additionally, the liposomal suspension may include lipid-protective agents which protect lipids against free-radical and lipid-peroxidative damages on storage. Lipophilic free-radical quenchers, such as alpha-tocopherol and water-soluble iron-specific chelators, such as ferrioxamine, are suitable. Leucovorin may also be administered with compositions underlying the invention through standard techniques to enhance the life span of administered fluoropyrimidines.

In addition to pharmaceutical compositions, suitable formulations for veterinary use may be prepared and administered in a manner suitable to the subject. Preferred veterinary subjects include mammalian species, for example, non-human primates, dogs, cats, cattle, horses, sheep, and domesticated fowl. Subjects may also include laboratory animals, for example, in particular, rats, rabbits, mice, and guinea pigs.

The following examples are offered to illustrate, but not to limit, the invention.

### EXAMPLES

### Example 1:

### Cetuximab® Enhances the Activity of Irinotecan, as well as Liposomal Irinotecan, in the DLD-1 Human Colon Xenograft Model

This example compares efficiency of comprising either free or liposomal camptothecin and an epidermal growth factor receptor inhibitor (*e.g*., Cetuximab®) compared to the individual agents. The efficacies of free irinotecan and free Cetuximab® were compared to the combination of the two and, similarly, the efficacies of liposomal irinotecan and free Cetuximab® were compared to the combination of these two agents.

Briefly, in order to perform tumor studies on mice, animals are inoculated subcutaneously with approximately 2 x 10⁶ tumor cells which are then allowed to grow to sufficient size before being treated. This is done using the methods described previously in PCT publication WO03/028696 (*supra*).

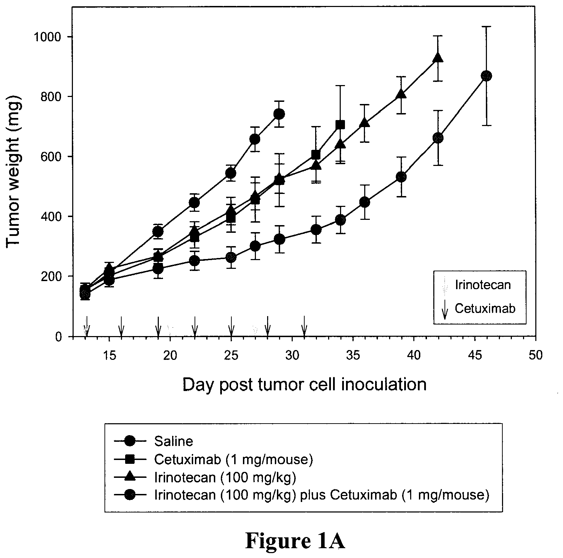

Either free irinotecan or Cetuximab® was administered to female nude-Foxnl mice at doses of 100 mg/kg or 1 mg/mouse, respectively on a multiple dosing schedule as shown by the arrows in Figure 1A. Cetuximab® was dosed on a Q3Dx7 schedule and irinotecan a Q7Dx3 schedule. The results show that the combination of both free agents is significantly improved compared to that of either agent alone.

Irinotecan was also actively loaded into DSPC/DSPG/Chol (70:20:10 mol ratio) liposomes. Lipid films were prepared by dissolving DSPC to 50 mg/ml, cholesterol to 50 mg/ml in chloroform, and DSPG to 25 mg/ml in chloroform/methanol/water (50/10/1). The lipids were then combined and, following solvent removal, the resulting lipid films were hydrated with a solution consisting of 100 mM Cu(gluconate)₂, 220 mM triethanolamine (TEA), pH 7.4 at 70°C. The resulting MLVs were extruded at 70°C to generate LUVs. The mean diameter of the resulting liposomes was determined by QELS (quasi-elastic light scattering) analysis to be approximately 100 nm +/- 20 nm. Subsequently, the liposomes were buffer exchanged into 300 mM sucrose, 20 mM Hepes, 30 mM EDTA (SHE), pH 7.4, using a hand-held tangential flow column and then into 150 mM NaCl, 20 mM Hepes (HBS), pH 7.4, thus removing any unencapsulated Cu(gluconate)₂. After loading and then cooling to room temperature, the samples were exchanged into saline (0.9% Sodium Chloride Injection, USP; pH 5.5, Baxter), by tangential flow dialysis to remove EDTA or unencapsulated drug(s). The extent of irinotecan loading was measured using absorbance at 370 nm against a standard curve. A drug to lipid ratio at each time point was generated using liquid scintillation counting to determine lipid concentrations (¹⁴C-CHE) concentrations.

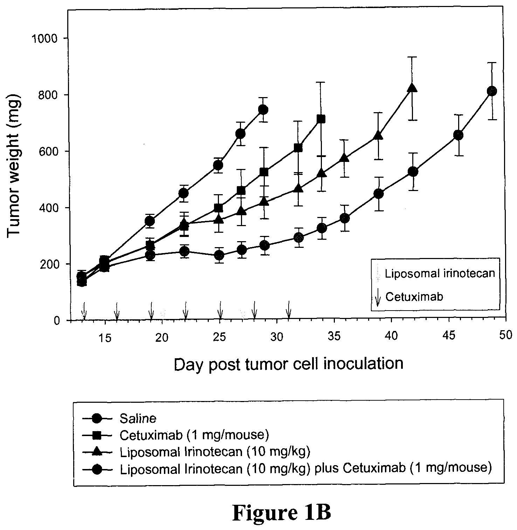

Either liposomal irinotecan or free Cetuximab® was administered to female nude-Foxnl mice at doses of 100 mg/kg or 1 mg/mouse, respectively on a multiple dosing schedule as shown by the arrows in Figure 1B. Cetuximab® was dosed on a Q3Dx7 schedule and liposomal irinotecan on a Q7Dx3 schedule. The results in Figure 1B show that liposomal irinotecan has greater activity than Cetuximab® alone and that the combination of both of these agents is significantly improved compared to that of either liposomal irinotecan or free Cetuximab® alone.

### Example 2:

### The Combination of CPX-1 and Avastin® is Additive Against the LS174T Human Colon Xenograft Model

In order to establish whether enhanced efficacy is observed in combinations of biological agents with two-drug liposomal compositions compared to either of these agents alone, the efficacies of dual-loaded liposomes in combination with the biological agent, Avastin®, were also compared to the therapeutic effects of Avastin® alone, as well as the dual-loaded liposomes alone. Avastin® is a monoclonal antibody against vascular endothelial growth factor (VEGF).

The present inventors have previously showed that the effect of combinations of camptothecins and fluoropyrimidines are ratio-dependent and that enhanced efficacies of combinations of these drug classes can occur when a ratio of the agents that gives at least an additive effect is maintained. In particular, a combination of the camptothecin, irinotecan, and the fluoropyrimidine, FUDR, was previously shown to exhibit a strong degree of synergy when the two agents are present at a 1:1 drug ratio. These two drugs can be co-loaded into liposomes which maintain the ratio after *in vivo* administration (a formulation termed "CPX-1") thereby delivering the drugs at the correct 1:1 ratio to the tumor site. Here the inventors determine if enhanced efficacy of CPX-1 occurs in the presence of Avastin® (also termed bevacizumab).

Irinotecan was actively loaded into DSPC/DSPG/Chol (70:20:10 mol ratio) liposomes containing passively entrapped FUDR. Lipid films were prepared by dissolving DSPC to 50 mg/ml, cholesterol to 50 mg/ml in chloroform, and DSPG to 25 mg/ml in chloroform/methanol/water (50/10/1). The lipids were then combined and following solvent removal the resulting lipid films were hydrated with a solution consisting of 100 mM Cu(gluconate)₂, 220 mM triethanolamine (TEA), pH 7.4 containing approximately 25 mg/ml FUDR (with trace amounts of ³H-FUDR) at 70°C. The resulting MLVs were extruded at 70°C to generate LUVs. The mean diameter of the resulting liposomes was determined by QELS (quasi-elastic light scattering) analysis to be approximately 100 nm +/- 20 nm. Subsequently, the liposomes were buffer exchanged into 300 mM sucrose, 20 mM Hepes, 30 mM EDTA (SHE), pH 7.4, using a hand-held tangential flow column and then into 150 mM NaCl, 20 mM Hepes (HBS), pH 7.4, thus removing any unencapsulated FUDR and Cu(gluconate)₂.

Irinotecan was added to these liposomes such that the FUDR to irinotecan mol ratio would be 1:1. Loading of irinotecan into the liposomes with an initial irinotecan to lipid ratio of 0.1:1 was facilitated by incubating the samples at 50°C for 10 minutes. After loading and then cooling to room temperature, the samples were exchanged into saline (0.9% Sodium Chloride Injection, USP; pH 5.5, Baxter), by tangential flow dialysis to remove EDTA or unencapsulated drug(s). The extent of irinotecan loading was measured using absorbance at 370 nm against a standard curve. A drug to lipid ratio at each time point was generated using liquid scintillation counting to determine lipid concentrations (¹⁴C-CHE) and FUDR concentrations (³H-FUDR).

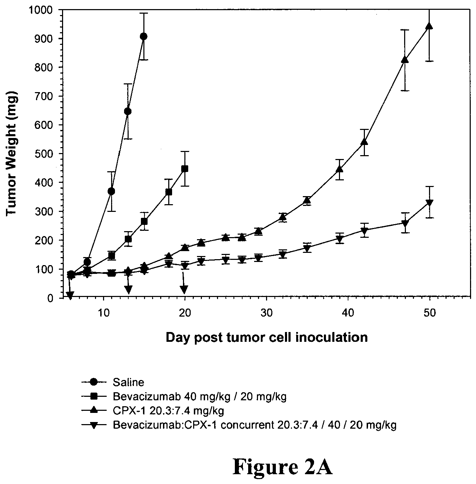

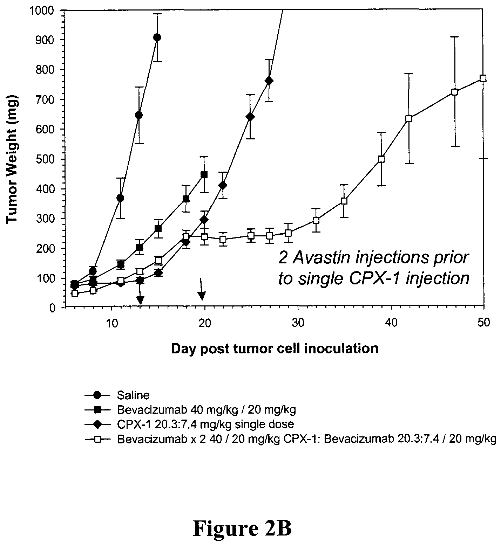

Either CPX-1 or free Avastin® was administered to female nude-Foxnl mice at doses detailed in Figures 2A and 2B, on a Q7Dx3 dosing schedule. The results in Figure 2A show that CPX-1 has greater activity than free bevacizumab and that the combination of both of these formulations is improved compared to that of either CPX-1 or bevacizumab alone.

This experiment was also repeated using mice that received pretreatment with bevacizumab (Avastin®). These mice received two injections of bevacizumab prior to a single injection of CPX-1. As seen in Figure 2B, the mice that were pretreated and received bevacizumab in combination with CPX-1 showed dramatic improvements in tumor reduction.

## Claims

1. A combination of
(a) first liposomes stably associated with at least one water-soluble camptothecin; and
(b) an antiangiogenic targeted antitumor agent which is a compound that decreases or inhibits the activity of an epidermal growth factor family receptor (EGFR) tyrosine kinase or that inhibits the activity of a vascular endothelial growth factor (VEGF) receptor tyrosine kinase or that binds to VEGF;
for use in treating a cancer in a subject.

2. A combination for use according to claim 1, wherein the antiangiogenic agent is an inhibitor of vascular endothelial growth factor (VEGF).

3. A combination for use according to claim 2, wherein the antiangiogenic agent is an antibody which binds to VEGF or inhibits a VEGF-receptor (VEGF-R).

4. A combination for use according to any of claims 1 - 3, wherein the water-soluble camptothecin is irinotecan, topotecan, 9-aminocamptothecin or lurtotecan.

5. A combination for use according to any of claims 1 - 4, wherein said first liposomes further comprise a fluoropyrimidine, wherein the mol ratio of said camptothecin to said fluoropyrimidine is non-antagonistic, and said camptothecin and fluoropyrimidine are stably associated with said first liposomes.

6. A combination for use according to any one of claims 1 - 4, wherein said combination further includes a fluoropyrimidine stably associated with second liposomes, wherein the mol ratio of said fluoropyrimidine and said water-soluble camptothecin is non-antagonistic, and the pharmacokinetics of said first and second liposomes are coordinated.

7. A combination for use according to claim 5 or 6, wherein the fluoropyrimidine agent is floxuridine, fluorouracil or UFT (tegafur/uracil).

8. A composition comprising
(a) liposomes associated with at least one water-soluble camptothecin; and
(b) an antiangiogenic targeted antitumor agent which is a compound that decreases or inhibits the activity of an epidermal growth factor family receptor (EGFR) tyrosine kinase or that inhibits the activity of a vascular endothelial growth factor (VEGF) receptor tyrosine kinase or that binds to VEGF;
for use in treating a cancer in a subject.

9. A composition for use according to claim 8, which composition further comprises liposomes associated with at least one fluoropyrimidine agent, wherein the mol ratio of said camptothecin and said fluoropyrimidine is non-antagonistic, said camptothecin and fluoropyrimidine are stably associated with said liposomes, and the pharmacokinetics of the liposomes are coordinated.

10. A composition for use according to claim 9, wherein said camptothecin and fluoropyrimidine are coencapsulated.

11. A composition for use according to any of claims 8 - 10, wherein the antiangiogenic agent is an inhibitor of vascular endothelial growth factor (VEGF).

12. A composition for use according to claim 11, wherein the antiangiogenic agent is an antibody which binds to VEGF or inhibits a VEGF-receptor (VEGF-R).

13. A composition for use according to any of claims 8 - 12, wherein the water-soluble camptothecin is irinotecan, topotecan, 9-aminocamptothecin or lurtotecan.

14. A composition for use according to any of claims 9 - 13, wherein the fluoropyrimidine agent is floxuridine, fluorouracil or UFT (tegafur/uracil).

15. A combination or composition for use according to any of claims 1 - 14, wherein said liposomes comprise distearoyl phosphatidylcholine (DSPC) or diarachidoyl phosphatidylcholine (DAPC) and distearoyl phosphatidylglycerol (DSPG) or dimyristoyl phosphatidylglycerol (DMPG) and less than 20 mol% cholesterol.

## Patentansprüche

1. Kombination von:
(a) ersten Liposomen, stabil verbunden mit mindestens einem wasserlöslichen Camptothecin; und
(b) einem antiangiogen gerichteten Antitumor-Agens, das eine Verbindung ist, die die Aktivität eines epidermalen Wachstumsfaktorfamilie-Rezeptors (EGFR) Tyrosinkinase vermindert oder inhibiert oder die die Aktivität eines vaskulären endothelialen Wachstumsfaktor (VEGF)-Rezeptors Tyrosinkinase inhibiert oder die an VEGF bindet;
zur Verwendung bei der Behandlung von Krebs bei einem Patienten.

2. Kombination zur Verwendung nach Anspruch 1, wobei das antiangiogene Agens ein Inhibitor von vaskulärem endothelialem Wachstumsfaktor (VEGF) ist.

3. Kombination zur Verwendung nach Anspruch 2, wobei das antiangiogene Agens ein Antikörper ist, der an VEGF bindet oder einen VEGF-Rezeptor (VEGF-R) inhibiert.

4. Kombination zur Verwendung nach einem der Ansprüche 1-3, wobei das wasserlösliche Camptothecin Irinotecan, Topotecan, 9-Aminocamptothecin oder Iurtotecan ist.

5. Kombination zur Verwendung nach einem der Ansprüche 1-4, wobei die ersten Liposome ferner ein Fluoropyrimidin umfassen, wobei das Mol-Verhältnis des Camptothecins zu dem Fluoropyrimidin nicht antagonistisch ist und das Camptothecin und Fluoropyrimidin stabil mit den ersten Liposomen verbunden sind.

6. Kombination zur Verwendung nach einem der Ansprüche 1-4, wobei die Kombination ferner ein Fluoropyrimidin umfasst, stabil verbunden mit zweiten Liposomen, wobei das Mol-Verhältnis des Fluoropyrimidins und des wasserlöslichen Camptothecins nicht antagonistisch ist und die Pharmakokinetiken der ersten und zweiten Liposome koordiniert sind.

7. Kombination zur Verwendung nach Anspruch 5 oder 6, wobei das Fluoropyrimidin-Agens Floxuridin, Fluoruracil oder UFT (Tegafur/Uracil) ist.

8. Zusammensetzung, umfassend
(a) Liposome, verbunden mit mindestens einem wasserlöslichen Camptotheccin, und
(b) einem antiangiogen gerichteten Antitumor-Agens, das eine Verbindung ist, die die Aktivität eines epidermalen Wachstumsfaktorfamilie-Rezeptors (EGFR) Tyrosinknase vermindert oder inhibiert oder die die Aktivität eines vaskulären endothelialen Wachstumsfaktor (VEGF)-Rezeptors Tyrosinkinase inhibiert, oder die an VEGF bindet;
zur Verwendung bei der Behandlung von Krebs bei einem Patienten.

9. Zusammensetzung zur Verwendung nach Anspruch 8, welche Zusammensetzung ferner Liposome umfasst, verbunden mit mindestens einem Fluoropyrimidin-Agens, wobei das Mol-Verhältnis des Camptothecins und des Fluoropyrimidins nicht antagonistisch ist, das Camptothecin und Fluoropyrimidin stabil mit den Liposomen verbunden sind und die Pharmkokinetiken der Liposome koordiniert sind.

10. Zusammensetzung zur Verwendung nach Anspruch 9, wobei das Camptothecin und Fluoropyrimidin co-eingekapselt sind.

11. Zusammensetzung zur Verwendung nach einem der Ansprüche 8-10, wobei das antiangiogene Agens ein Inhibitor von vaskulärem endothelialem Wachstumsfaktor (VEGF) ist.

12. Zusammensetzung zur Verwendung nach Anspruch 11, wobei das antiangiogene Agens ein Antikörper ist, der an VEGF bindet oder einen VEGF-Rezeptor (VEGF-R) inhibiert.

13. Zusammensetzung zur Verwendung nach einem der Ansprüche 8-12, wobei das wasserlösliche Camptothecin Irinotecan, Topotecan, 9-Aminocamptothecin oder Iurtotecan ist.

14. Zusammensetzung zur Verwendung nach einem der Ansprüche 9-13, wobei das Fluoropyrimidin-Agens Floxuridin, Fluorouracil oder UFT (Tegafur/Uracil) ist.

15. Kombination oder Zusammensetzung zur Verwendung nach einem der Ansprüche 1-14, wobei die Liposome Distearoylphosphatidylcholin (DSPC) oder Diarachidoylphosphatidylcholin (DAPC) und Disteearoylphosphatidylglycerol (DSPG) oder Dimyristoylphopsphatidylglycerol (DMPG) und weniger als 20 Mol % Cholesterin umfassen.

## Revendications

1. Combinaison de :
(a) premiers liposomes qui sont associés de façon stable avec au moins une camptothécine soluble dans l'eau ; et
(b) un agent antitumoral ciblé antiangiogénique qui est un composé qui diminue ou inhibe l'activité d'une tyrosine kinase de récepteur de la famille du facteur de croissance épidermique (EGFR) ou qui inhibe l'activité d'une tyrosine kinase de récepteur du facteur de croissance endothéliale vasculaire (VEGF) ou qui se lie sur le VEGF ;
pour une utilisation dans le traitement d'un cancer chez un sujet.

2. Combinaison pour une utilisation selon la revendication 1, dans laquelle l'agent antiangiogénique est un inhibiteur du facteur de croissance endothéliale vasculaire (VEGF).

3. Combinaison pour une utilisation selon la revendication 2, dans laquelle l'agent antiangiogénique est un anticorps qui se lie sur le VEGF ou qui inhibe un récepteur de VEGF (VEGF-R).

4. Combinaison pour une utilisation selon l'une quelconque des revendications 1 à 3, dans laquelle la camptothécine soluble dans l'eau est l'irinotécan, le topotécan, la 9-aminocamptothécine ou le lurtotécan.

5. Combinaison pour une utilisation selon l'une quelconque des revendications 1 à 4, dans laquelle lesdits premiers liposomes comprennent en outre une fluoropyrimidine, dans laquelle le rapport molaire de ladite camptothécine sur ladite fluoropyrimidine est non antagoniste, et lesdites camptothécine et fluoropyrimidine sont associées de façon stable avec lesdits premiers liposomes.

6. Combinaison pour une utilisation selon l'une quelconque des revendications 1 à 4, dans laquelle ladite combinaison inclut en outre une fluoropyrimidine qui est associée de façon stable avec des seconds liposomes, dans laquelle le rapport molaire de ladite fluoropyrimidine et de ladite camptothécine soluble dans l'eau est non antagoniste, et les pharmacocinétiques desdits premiers et seconds liposomes sont coordonnées.

7. Combinaison pour une utilisation selon la revendication 5 ou 6, dans laquelle l'agent fluoropyrimidine est le floxuridine, le fluorouracile ou l'UFT (tégafur/uracile).

8. Composition comprenant :
(a) des liposomes qui sont associés avec au moins une camptothécine soluble dans l'eau ; et
(b) un agent antitumoral ciblé antiangiogénique qui est un composé qui diminue ou inhibe l'activité d'une tyrosine kinase de récepteur de la famille du facteur de croissance épidermique (EGFR) ou qui inhibe l'activité d'une tyrosine kinase de récepteur du facteur de croissance endothéliale vasculaire (VEGF) ou qui se lie sur le VEGF ;
pour une utilisation dans le traitement d'un cancer chez un sujet.

9. Composition pour une utilisation selon la revendication 8, laquelle composition comprend en outre des liposomes qui sont associés avec au moins un agent fluoropyrimidine, dans laquelle le rapport molaire de ladite camptothécine sur ladite fluoropyrimidine est non antagoniste, et lesdites camptothécine et fluoropyrimidine sont associées de façon stable avec lesdits liposomes, et les pharmacocinétiques des liposomes sont coordonnées.

10. Composition pour une utilisation selon la revendication 9, dans laquelle lesdites camptothécine et fluoropyrimidine sont co-encapsulées.

11. Composition pour une utilisation selon l'une quelconque des revendications 8 à 10, dans laquelle l'agent antiangiogénique est un inhibiteur du facteur de croissance endothéliale vasculaire (VEGF).

12. Composition pour une utilisation selon la revendication 11, dans laquelle l'agent antiangiogénique est un anticorps qui se lie sur le VEGF ou qui inhibe un récepteur de VEGF (VEGF-R).

13. Composition pour une utilisation selon l'une quelconque des revendications 8 à 12, dans laquelle la camptothécine soluble dans l'eau est l'irinotécan, le topotécan, la 9-aminocamptothécine ou le lurtotécan.

14. Composition pour une utilisation selon l'une quelconque des revendications 9 à 13, dans laquelle l'agent fluoropyrimidine est le floxuridine, le fluorouracile ou l'UFT (tégafur/uracile).

15. Combinaison ou composition pour une utilisation selon l'une quelconque des revendications 1 à 14, dans laquelle lesdits liposomes comprennent de la distéaroyl phosphatidylcholine (DSPC) ou de la diarachidoyl phosphatidylcholine (DAPC) et du distéaroyl phosphatidylglycérol (DSPG) ou du dimyristoyl phosphatidylglycérol (DMPG) et moins de 20 % mol de cholestérol.
